# EUROPEAN PATENT APPLICATION

(11) **EP 4 498 381 A1**
(43) Date of publication of application: **29.01.2025**
(21) Application number: 23187849.7
(22) Date of filing: 26.07.2023
(51) Int. Cl.: G16H 40/20, G06N 3/02, G06N 20/00, G16H 50/30

(54) **CLINICAL DECISION SUPPORT SYSTEM**

(71) Applicant: Koninklijke Philips N.V., 5656 AG Eindhoven (NL)
(72) Inventor: BOUTS, Mark Jacobus Rosalie Joseph, 5656AG Eindhoven (NL); BULUT, Murtaza, 5656AG Eindhoven (NL); VAN LIESHOUT, Ron Martinus Laurentius, 5656AG Eindhoven (NL); HUIJBREGTS, Laurentia Johanna, 5656AG Eindhoven (NL)
(74) Representative: Philips Intellectual Property & Standards

(57) **Abstract**

A clinical decision support system including an orchestrator module adapted to coordinate the triggering of inference algorithms and the execution of clinical protocols. The system further includes modules for checking input requirements for inference algorithms are met before an algorithm is released for use.

## Description

### FIELD OF THE INVENTION

This invention relates to a clinical decision support system guided by patient monitoring data.

### BACKGROUND OF THE INVENTION

The intensive care unit (ICU) is a complex and changeable environment which requires dedicated attention to effectively treat a critically ill patient. For patient treatment, health care practitioners (HCP) may utilize a variety of monitoring and life supporting equipment. This equipment generates a large quantity of data, and the interpretation of this data including decisions as to appropriate response actions, requires specialized training and experience.

The data and resultant action are evaluated by a multidisciplinary team that includes nurses, physician-assistants, intensivists, nutritionists, and others. The team works continuously and intensively for the well-being of the patient. Such teams implement rules and systems for collaboration and effective communication, to streamline the clinical workflow and to maximize patient comfort and outcome. However, demands on ICU personnel are high, with many staff facing burn-out. There is therefore a need for tailored ICU workflow support that might relieve part of the (cognitive) burden of patient monitoring and care, while retaining full clinician control and oversight.

Standardization of care through, for example, guidelines, protocols, and standard operating procedures (SOPs) have been proposed as a way of better supporting workflow. However, the success of standardization is dependent upon the adoption of protocols. Understanding when and for what specific patient population a protocol should be used is important. Using the incorrect protocol for an alternate patient population may have detrimental consequences on the well-being and recovery of the patient. Currently, standardization is cumbersome. Protocols and guidelines are rarely digitized, are very static, and lack personalization. Guidelines may provide a general guide as to which steps or actions should be conducted during patient management but leave considerable room for interpretation. Identifying the appropriate concrete steps for the current or imminent situation, collecting the appropriate information, and interpreting that information to identify the next steps is cognitively demanding, timeconsuming, and error prone. As a result, these steps become workflow disruptive rather than supportive and therefore challenge its adoption.

A growing area of interest is the use of artificial intelligence (AI) based inference algorithms for providing support in the monitoring, diagnosis, prognosis, and treatment of patients. Typically, such algorithms serve a specific purpose. For example, algorithms may predict an imminent need for a particular treatment, predict a risk of one or more complications, or may assess a possibility of adjusting a patient management regime (e.g. ICU discharge readiness, ventilator weaning readiness). In addition to serving a particular purpose, inference algorithms are typically trained for use in respect of a particular patient population (e.g. neuro, post-cardiac surgery, non-trauma patients). Thus, given the large number of different possible purposes and different possible patient populations, the number of algorithms can become very large. It may thus become increasingly complex to understand the purpose of each individual algorithm, the circumstances under which each algorithm should or should not be used, how information output from an algorithm should be interpreted, and how the algorithms should interact.

Algorithms may be in some cases workflow disruptive. For example, some algorithms may be adapted to compute a risk parameter and may require a certain set of data as input. This set of input data may be broader than the set of data that is actually available at any given moment in an ICU. For example, it may include, in addition to vital sign data, laboratory results, ventilation information and patient demographics. Adding these data types is not straightforward and requires time. Consequently, paradoxically, the increased sophistication of risk prediction offered by intelligent algorithms can bias against their adoption, since the staff time and resource needed to provide all necessary inputs may outweigh the benefits of their use in clinical practice.

Furthermore, in a case where a multiplicity of monitoring algorithms are to be used, computational demand is increased. For example, a system comprising multiple algorithms monitoring multiple ICU patients, and utilizing many different types of input information, poses a challenge in terms of the implementing software.

For example, to address a certain clinical scenario in an ICU environment, an HCP may need to have instantaneous access to various types of information. Delays due to network lag or computational over-demand are clinically unacceptable.

Furthermore, where a larger number of different algorithms are provided, managing supply of input information to the different algorithms may be a challenge. Some algorithms may be missing certain information and some algorithms may require the same input information and thus there is the potential for conflict or competition between algorithms for resources. This poses a challenge in terms of control and management.

Thus, in summary, an increasing number of algorithms and dynamic protocols may pose additional technical challenges when being implemented in practice. In particular, controlling timing of activation of the different algorithms is a challenge. For example, operating and maintaining all algorithms simultaneously compromises decision-making and action traceability. Especially when misdiagnosis or medical errors are suspected, understanding actions and their triggers is important for follow-up. Furthermore, algorithms and protocols may interfere or complete, thereby compromising effectiveness, creating confusion, and possibly undermining correct decision-making. Furthermore, keeping track of the multiplicity of algorithms may pose additional cognitive burden to healthcare providers. For instance, a user would need to keep track of the various requirements for execution of each algorithm and protocol, including data input requirements and patient population requirements. In a cognitively demanding environment, such as an ICU, keeping track of these conditions imposes cognitive demand on HCPs, thereby increasing risk of oversight or error and thus patient harm.

### SUMMARY OF THE INVENTION

The invention is defined by the claims.

According to examples in accordance with an aspect of the invention, there is provided a clinical decision support system.

The system comprises an algorithm datastore storing: a plurality of inference algorithms, wherein each inference algorithm is adapted to receive a set of input patient data and generate an output metric or score indicative of a pre-defined aspect of patient status or for a pre-defined clinical risk.

The system further comprises a protocol datastore storing: a plurality of clinical protocol specifications, each protocol specification comprising a data representation of a clinical protocol associated with monitoring and/or treating a patient in respect of a pre-defined clinical condition or clinical risk. Each protocol specification may optionally include one or more machine-executable steps.

The system further comprises an algorithm monitor module adapted to: receive a request message for access to one of the inference algorithms, assess a predefined one or more release conditions for the inference algorithm, and generate a release flag/trigger for a given algorithm if the predefined one or more release conditions are met.

The system further comprises a protocol module adapted to: receive a request message for preloading one of the clinical protocols, and generate a protocol loading package comprising the protocol specification for the specified protocol.

The system further comprises a risk database, recording, for each of a plurality of different clinical risks, one or more suitable protocols and/or inference algorithms for responding to the risk.

The system further comprises an orchestrator module, adapted to: receive one or more risk messages or alerts indicative of possible clinical risks; responsive to receiving a risk message, consult the risk database to identify an inference algorithm in the algorithm datastore suitable for monitoring the relevant risk and/or to identify a protocol in the protocol datastore suitable for managing the risk; and generate a request message for sending to the algorithm monitor module for requesting access to the identified inference algorithm(s) and/or sending a request message to the protocol module for requesting loading or pre-loading of the identified protocol.

Thus it is proposed according to embodiments of the present invention to provide a system for facilitating intelligent interaction between AI monitoring algorithms and clinical protocols, via an orchestration module which is adapted to control when to load and push algorithms or protocols to hosting platforms. To facilitate this, the orchestration module receives alerts or messages indicating potential risks (e.g. a patient monitoring station where the patient is admitted, from a monitoring algorithm exceeding a trigger threshold, or from a running protocol), and, responsive thereto, looks up in a database or table an appropriate one or more algorithms or protocols to load/trigger in response to the potential risk. This functionality allows AI algorithms to trigger suitable protocols or other algorithms, and allows protocols to trigger suitable algorithms or other protocols. All of this is done at least semiautonomously, without placing cognitive burden on a healthcare provider.

In some embodiments, some or all of the inference algorithms may be machine learning algorithms.

Executing one of the algorithms may comprise running the algorithm continuously by supplying the algorithm a real-time stream of input patient data and recording a data series of output values from the algorithm. In this way, each risk prediction algorithm can be used to monitor a given risk, and to detect when a risk exceeds one or more threshold boundaries.

At least a subset of the inference algorithms may be predictive algorithms, for example adapted to predict probability of an adverse outcome or event. For example, at least a subset of the inference algorithms may be adapted to generate an output risk score indicative of a risk of an adverse event or outcome. At least some of the algorithms may be adapted to receive a set of input data and generate a stability index or score indicative of a stability of certain physiological system, such as hemodynamic stability.

The system may further comprise a patient clinical data source which may provide a source of input patient data for the inference algorithms when these are running and for the protocols in the cases where a protocol utilizes patient data to run. The patient data source may for example comprise a patient monitoring subsystem which may comprise one or more measurement devices for measuring clinical parameters, e.g. physiological parameters of the patient.

In this disclosure, the term `clinical protocol' is intended to cover and encompass any standardized plan or guideline or procedure or workflow within a clinical context which details a standardized set of steps or processes to be performed in a certain clinical context. For example, using the terminology often used in the field, for the present disclosure, 'protocol' is intended to cover and encompass any of: standard operating procedures (SOPs), Guidelines, and clinical protocols.

In some embodiments, the system further comprises an algorithm hosting platform adapted to host and run any of the one or more of the inference algorithms.

In some embodiments, the orchestrator module may be adapted to: receive release flags generated by the algorithm monitoring module responsive to a sent request message relating to a specified algorithm; and push released algorithms to the algorithm hosting platform for execution.

Thus, the algorithm monitoring module is adapted to monitor and manage the availability of inference algorithms by generating release flags when conditions for algorithm execution are met, and the orchestrator module is adapted to control execution of algorithms based on the presence or absence of such release flags. Thus, the requirement to monitor whether conditions for executing a certain algorithm are met is removed from the demands of the caregiver.

In some embodiments, the system further comprises a protocol hosting platform adapted to host any of the one or more protocols.

In some embodiments, the orchestrator module is adapted to: receive protocol loading packages from the protocol module, and push protocol specifications comprised by received protocol loading packages to the protocol hosting platform.

Thus, the protocol module is adapted to generate protocol loading packages, wherein the protocol hosting platform is adapted to use the loading package to host the protocol. Hosting of a protocol may comprise presenting prompts on a user interface, e.g. displaying a document which lists details of steps of the protocol in accordance with the protocol specification or automatically displaying a next step in the protocol. Additionally or alternatively, the hosting of a protocol may comprise automatically executing one or more machine-executable steps of the protocol.. Additionally or alternatively, the hosting of a protocol may comprise tracking execution of steps of a protocol.

In some embodiments, the orchestrator module is adapted to receive the aforementioned risk messages or alerts from any one or more of: a patient admissions system, the algorithm hosting platform, the protocol hosting platform, the protocol module and/or a user interface.

In some embodiments, each inference algorithm is adapted to generate a risk alert responsive to the metric or score of the algorithm passing a risk threshold. This may then trigger the orchestrator module to take one or more response actions, wherein the one or more response actions may include requesting a further one or more algorithms from the algorithm monitor module, and/or may include requesting loading or pre-loading of a further one or more clinical protocols.

In some embodiments, the protocol datastore further stores, for each protocol, metadata indicating: any further clinical risk associated with any one or more steps of the protocol. Responsive to receipt of a request message, the protocol module may be further adapted to read the metadata for the specified protocol to identify any further clinical risk associated with any one or more steps of the protocol. The protocol loading package may further include the retrieved further clinical risk information associated with the relevant protocol.

In some embodiments, the protocol datastore further stores, for each protocol, metadata indicating: required patient personalization data for configuring the protocol.

In some embodiments, responsive to receipt of a request message, the protocol module may be further adapted to: read the metadata for the specified protocol to identify the required patient personalization data; access a patient data source to obtain the required patient personalization data; and configure the protocol in the protocol package with the patient personalization data.

Thus, in some embodiments, the protocol module is configured to automatically manage the retrieval of necessary data for configuring or tuning parameters of a protocol in accordance with patient-specific data. This relieves the clinician of the burden of keeping track of needed personalization data, of retrieving the data and of adjusting the protocol in accordance with the data.

Examples of personalization data may include for example any one or more of: patient demographic information, patient medical history, comorbidities, past interventions, vital signs, predictive algorithm outputs, alarm settings (e.g., an upper and/or lower alarm threshold, e.g. 90 mmHg<SBP< 140 mmHg, SpO2>94%), any score or probability related to high risk of death (e.g. GCS, APACHEII, SAPSII, SOFA), ICU mortality, hospital mortality, 90 days mortality or ICU length of stay.

The patient data source may be a patient medical record database. The patient data source may be a patient monitoring system.

For example, for at least a subset of the protocols stored in the protocol datastore, one or more steps of the protocol may be configurable in dependence upon values for one or more elements of patient personalization data. The protocol module may be adapted to configure the one or more configurable steps of the protocol in the protocol loading package with the patient data. For example, the protocol may include a branching or conditional logic structure, wherein the rules or thresholds defining the branching are variable based on patient characteristics, such as age, weight, gender and other demographics.

In some embodiments, the protocol datastore further stores, for each protocol, metadata indicating: required patient clinical parameter data for running the protocol. Responsive to receipt of a request message, the protocol module may be further adapted to: read the metadata for the specified protocol to identify the required clinical parameter data; determine whether the required clinical parameter data for the specified protocol is currently available; and if the required clinical parameter data is not available, execute a data collection sub-routine for obtaining the required clinical parameter data. For example, determining whether the required clinical parameter data is currently available may comprise determining what data is available from a patient clinical data source for the system, e.g. a patient monitoring subsystem.

In some embodiments, the data collection sub-routine may include: sending to the orchestrator module a data collection request message including an indication of recommended supplementary data to be acquired to supplement the available patient clinical parameter data; and wherein the orchestrator module is adapted to communicate with a data collection sub-system to request acquisition of the supplementary data. For example, the data collection subsystem may be a patient monitoring sub-system. Optionally, the data collection sub-routine may include generating a prompt message at the user interface to prompt a user to take steps to enable the supplementary data to be collected, for example by attaching or installing a measurement device to a patient and linking it with a patient monitoring subsystem.

In some embodiments, the protocol module is adapted to execute background operations during running of the system comprising, for at least a subset of the protocols in the protocol datastore, monitoring whether the required clinical parameter data for running each protocol is currently available and, if the required clinical parameter data is not available, executing a data collection subroutine for acquiring supplementary data.

In some embodiments, the algorithm datastore further stores, for each inference algorithm, metadata indicating required input data for running the algorithm. This may for example include required patient sensor data, e.g. physiological parameter data. This might include one or more vital signs for example. Additionally or alternatively, it may include required scan or image data, and/or may include diagnostic data.

In some embodiments, responsive to receipt of a request message relating to a specified algorithm, the algorithm monitor module may be further adapted to: read the metadata for the relevant specified algorithm in the algorithm datastore; determine whether the required input data for the algorithm is currently available; and generate the release flag/trigger for the specified algorithm only if the input data requirements are met. In other words, the previously mentioned release conditions may include the input data requirements being met. For example, determining whether the required input data is currently available may comprise determining whether the data is available from a patient clinical data source for the system, e.g. a patient monitoring subsystem.

Thus, according to this set of embodiments, the algorithm monitor module automatically keeps track of required input information for an algorithm to run correctly, and restricts usage of algorithms if the necessary input information is not available. This prevents algorithms from being run in the absence of all necessary information (which could lead to generation of false and misleading results). It also eliminates a need for the clinician to check data input requirements for each algorithm.

In some embodiments, the input data requirements are not met, the algorithm monitor module may be further adapted to execute a data collection sub-routine for acquiring supplementary data to supplement the currently available patient data.

For example, in some embodiments, the data collection sub-routine includes: sending to the orchestrator module a data collection request message including an indication of recommended supplementary data to be acquired to supplement the currently available patient data. The orchestrator module may be adapted to communicate with a data collection sub-system, e.g. patient monitoring subsystem, to request acquisition of the supplementary data.

Additionally or alternatively, in some embodiments, the data collection sub-routine includes: sending a protocol recommendation message to the orchestrator module recommending execution of a protocol. The recommended protocol may include steps for acquiring at least a subset of the required input information for the specified algorithm which is currently not available.

In some embodiments, the algorithm monitor module is adapted to execute background operations during running of the system comprising, for at least a subset of the algorithms in the algorithm datastore, monitoring whether the required input data for each algorithm is currently available and, if the required input data is not available, executing a data collection sub-routine for acquiring supplementary data to supplement the input patient data. Thus, in this way, by the time an algorithm comes to be executed, all necessary data may be available for its execution. This avoids any delay between request for access to an algorithm and its execution.

In some embodiments, the algorithm datastore may further store, for each inference algorithm, associated metadata including a patient population type for which the algorithm is configured (e.g. trained in the case of a machine learning algorithm) to be applied. Responsive to a request message, the algorithm monitor module may be adapted to: read the metadata for the algorithm in the algorithm datastore; determine whether a patient associated with the input patient data matches the patient population type; and generate a release flag/trigger for a given algorithm only if the patient population type requirements are met. In some embodiments, the input patient data may include an indication of a patient population type to which a patient belongs, and wherein the determining whether a patient associated with the input patient data matches the patient population type includes referencing the input patient data. This avoids an algorithm being executed for monitoring risk for a patient for whom the algorithm has not been designed or trained. This avoids potentially generating false or misleading results.

In some embodiments, the algorithm datastore further stores, for each of at least a subset of the inference algorithms, metadata which includes an indication of an optimal timing for starting and/or stopping the algorithm. The orchestrator module may be further adapted to control timing of pushing each of the at least subset of algorithms in dependence upon the optimal timing information in the metadata for the respective algorithm.

In some embodiments, the orchestrator module is further adapted to: receive a prescription medication recommendation from a running protocol and fill one or more fields of a medication request form based on the request. The orchestrator module may in some embodiments further generate an alert on a user interface to alert a user to the medication recommendation. The orchestrator module may further receive a user input from the user interface indicative of acceptance or rejection of the medication recommendation, and responsive to acceptance, forward the form to a prescription request platform.

In some embodiments, the system may further include a patient monitoring sub-system comprising one or more physiological parameter sensors and a patient monitoring control module adapted to receive and compile data from the physiological parameter sensors.

Another aspect of the invention is a computer-implemented method comprising performing steps carried out by any system described in any embodiment or example in this disclosure, or in accordance with any claim of this application. Another aspect of the invention is a computer program product comprising computer program code configured, when run on a processor, to cause the processor to perform such a method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of an example system in accordance with one or more embodiments of the invention;
Fig. 2 is a graph indicating complication risk as a function of time for an example scenario;
Fig. 3 is a block diagram of an example system, illustrating an example implementation scenario for the system;
Fig. 4 shows a set of graphs showing complication risk for a set of adverse events as a function of time for an example scenario;
Fig. 5 shows a block diagram of an example system, illustrating an example implementation scenario for the system;
Fig. 6 shows a set of graphs showing complication risk for a set of adverse events as a function of time for an example scenario; and
Fig. 7 shows a block diagram of an example system, illustrating an example implementation scenario for the system.

### DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a clinical decision support system including an orchestrator module adapted to co-ordinate the triggering of inference algorithms and the execution of clinical protocols. The system further includes modules for checking input requirements for inference algorithms are met before an algorithm is released for use.

An aim according to embodiments of the present invention is to support a clinical workflow while keeping workflow disruptions at a minimum. Standardization of care through the use of protocols has been proposed as a way of improving clinical healthcare. AI-based algorithms have been proposed for support in alerting, diagnosis, prognosis, or treatment. While various clinical decision support tools have been proposed which have a theoretical benefit, in real-world scenarios, it can be challenging for a care giver to determine the particular circumstances under which each tool (e.g. algorithms, protocols) is appropriate for use. The typically large body of algorithms and protocols available within a specific setting can often simply increase complexity of care provision. In many cases, it can be impossible within a short time window available to select the most appropriate decision support tool, to check its data requirements are met, and to load it to the correct hosting platform. Thus, many tools are simply impractical for real world use. While, in theory, smart predictive alarming may increase the window of opportunity for follow-up or adaptation through evidence-based protocols or algorithms, having the right actionable guidance available at the right moment is challenging.

At least a subset of embodiments of the present invention propose a system configured for managing clinical decision support tools, including monitoring algorithms and clinical protocols, and organizing the selection of a most appropriate tool given a certain clinical scenario. Some embodiments further check that data requirements for execution of algorithms or clinical protocols are met.

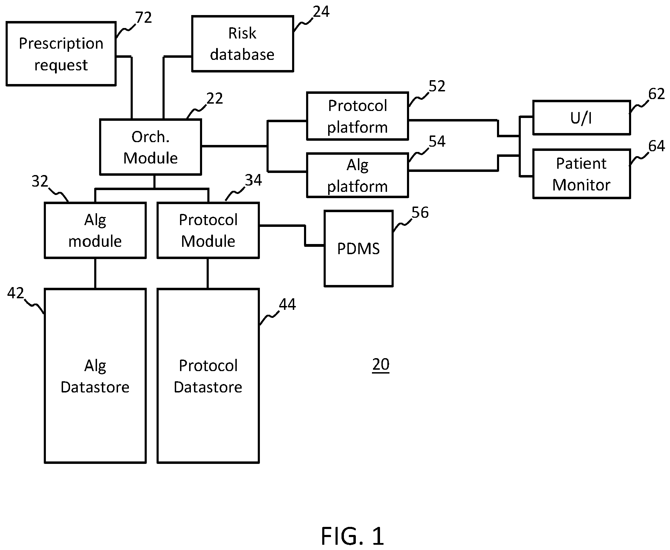

Fig. 1 outlines in block diagram form functional components of an example system 20 in accordance with one or more embodiments. The components will be recited in summary, before being explained further in the form of example embodiments.

The system 30 includes an algorithm datastore 42 storing: a plurality of algorithms for use in monitoring patient condition and/or predicting patient condition. At least a subset of the algorithms may be machine learning algorithms. The algorithms may be inference algorithms. At least some of the algorithms may be prediction/predictive algorithms. For example, this may mean that the algorithms may be configured to infer a risk of a certain adverse event or outcome. For example, the predictive algorithms may be risk prediction algorithms. Each algorithm may be configured to receive a set of input patient data and generate a risk score for a pre-defined clinical risk. The pre-defined clinical risk may be risk of occurrence of a certain adverse event or outcome. At least some of the algorithms may be adapted to receive a set of input data and generate a stability index or score indicative of a stability of certain physiological system, such as hemodynamic stability, and wherein if the stability index falls below a certain level, this indicates an adverse patient status. In other words, more generally, each algorithm may be adapted to receive a set of input patient data and generate an output metric or score indicative of a predefined aspect of patient status or for a pre-defined clinical risk.

The system 20 further includes a protocol datastore 44 storing: a plurality of clinical protocol specifications, each protocol specification comprising a data representation of a clinical protocol or workflow associated with monitoring and/or treating a patient in respect of a pre-defined clinical condition or clinical risk. The protocol specification may include specification of one or more machine-executable steps. The protocol specification may include specification of one or more human-executable steps.

The system 20 further includes an algorithm monitor module 32 adapted to: receive request messages for access to any one of the inference algorithms, assess a predefined one or more release conditions for the inference algorithm, and generate a release flag/trigger for a given algorithm if the predefined one or more release conditions are met.

The system 20 further includes a protocol module 34 adapted to: receive a request message for loading or preloading one of the protocols, and generate a protocol loading package comprising the protocol specification for the specified protocol.

The system 20 further includes a risk database 24, recording, for each of a plurality of different clinical risks, one or more suitable clinical protocols and/or inference algorithms for responding to the risk. In some embodiments, the risk database could take the form of a simple lookup table that associates different possible risk alerts or messages with one or more appropriate algorithms or protocols for monitoring or managing the risk. Additionally or alternatively, the risk database may include sets of rules or logic which define appropriate responses to different risk messages or alerts, or other system triggers received at the orchestrator module. These rules may include conditional logic, in which certain steps are implemented or not conditionally on a short-term history of steps already performed during a monitoring session for a patient.

The system 20 further comprises an orchestrator module 22. The orchestrator module is adapted to: receive one or more risk messages or alerts indicative of possible clinical risks; responsive to receiving a risk message, consult the risk database 24 to identify an inference algorithm in the algorithm datastore 42 suitable for monitoring the relevant risk and/or to identify a protocol in the protocol datastore 44 suitable for managing the risk. The orchestrator module 22 is further adapted to generate a request message for sending to the algorithm monitor module 32 for requesting access to the identified risk monitoring algorithm(s) and/or sending a request message to the protocol module 34 for requesting loading or pre-loading of the identified protocol.

It is noted that although the various modules and other components in Fig. 1 are shown illustratively as separate entities or units within the system 30, this is schematic only. In practice, these could simply be software modules which may all be encoded in programming of a single processor, or their functions may be performed in a distributed way by a plurality of processors. In practice, the functionality of the various modules and other components could be encoded on a same set of one or more processors.

In the illustrated example of Fig. 1, the system 30 further comprises a user interface 62. For example, this may be console or control module, for instance with a display and user input device, for use by a clinician user in monitoring the operations of the system, and in particular the orchestration module 22. The user interface may be used by the protocol hosting platform to display information relating to any protocols being executed. This may include for example a presentation of a document which contains a text representation of a given protocol specification. It may comprise one or more status indicators indicating a stage within a protocol which is currently active.

In the illustrated example of Fig. 1, the system 30 further comprises a patient monitoring sub-system 64. This may comprise one or more patient monitoring sensors, e.g. physiological parameter sensors, for acquiring input data for supplying to one or more of the inference algorithms and/or one or more of the protocols. It may further comprise a patient monitoring control module adapted to receive and compile data from the sensors.

In the illustrated example of Fig. 1, the system further comprises a Patient Data Management System (PDMS). This may store patient data for each of a plurality of patients, wherein each data record for a patient may include one or more of: patient demographics, medical history, lab results, medication records, vital signs (e.g. heart rate, blood pressure, temperature, and/or respiratory rate); clinical notes, and/or treatment plans.

In the illustrated example of Fig. 1, the system 30 further comprises an ordering platform 72, which is adapted to receive prescription requests or other orders (e.g. diagnostics) and to communicate with an external fulfilment system for fulfilling the orders. For example, in some embodiments, the orchestrator module 22 is further adapted to: receive a prescription medication recommendation from a running protocol and fill one or more fields of a medication request form based on the request. In some embodiments, the orchestrator module 22 may generate an alert at the user interface 62 to alert a user to the medication recommendation, receive a user input from the user interface indicative of acceptance or rejection of the medication recommendation, and responsive to acceptance, forward the form to the ordering platform 72.

Additionally or alternatively, orders could relate to laboratory tests, or diagnostics (e.g. CT, Ultrasound).

A user may fulfil an order by updating the order and submitting it. Alternatively, a user can ignore the order. The order might be automatically removed after a pre-defined time (e.g. 1 hour).

In the illustrated example of Fig. 1, the system 30 further comprises an algorithm hosting platform 54 adapted to host and run any of the inference algorithms. For example, this may provide a runtime environment for execution of the algorithms. This could be achieved using containerization technologies, which can provide isolated and consistent environments for running code. It may include input/output management, permitting continuous real-time input of data for feeding to running algorithms, such as sensor data (e.g. physiological parameter sensor data), scan data, patient demographic data. This may also manage output of information, for example for communicating outputs of running algorithms to a user interface for presentation to a user, and/or communication to a datastore, such as a PDMS.

In the illustrated example of Fig. 1, the system further comprises a protocol hosting platform 52 adapted to host any of the one or more protocols. As mentioned above, a protocol is associated with a protocol specification which lists a sequence of steps or actions or monitoring operations to be performed for managing a certain risk or a certain scenario. In a most simple example, hosting of a protocol may comprise simply presenting a human-readable representation of the protocol specification on a display of a user interface, so that a clinician user can implement the protocol. For example, in a simplest case, the protocol hosting platform may be configured to generate a GUI output using a display of the user interface, wherein the content of the GUI output is determined based on the protocol specification.

In some embodiments, the protocol hosting platform is adapted to keep track of which steps of the protocol are being applied, and which steps have already been applied. This may be useful since completion of certain steps may act as system triggers for starting or stopping other protocols or one or more of the inference algorithms. In a simple case, a human clinician user could provide input at the user interface to indicate when a given step of a protocol has been completed. A running record may be kept for each running protocol indicative of which steps of the protocol have been completed. Additionally, implementation details associated with the running of a protocol may be recorded and tracked such as the input data used, the output generated, time of implementation, and records generated such as EMR entries and clinician notes. Thus, in summary, the protocol hosting platform may include a tracking mechanism for tracking in a quantitative manner how a particular protocol is applied. This information may optionally then be used by the orchestrator module 22 to determine suitable algorithms, or suitable protocols to start or stop.

In some embodiments, the protocol hosting platform may be adapted to control automatic execution of at least a subset of the steps of a given protocol. For example, some of the steps detailed by the protocol specification may be machine-executable steps, or may trigger machine executed steps.

For example, a certain step of the protocol specification may indicate that a particular clinical risk should be monitored. Responsive to an indication in a protocol specification to monitor a defined clinical risk, a risk message may be communicated to the orchestrator module by the protocol hosting platform. The orchestrator module may then handle the risk message by consulting the risk database 24 to determine any appropriate protocol or algorithm for managing or monitoring the risk.

By way of a further example, a protocol specification may indicate that execution of a specific inference algorithm is needed. Thus, the optional machine-executable steps of the protocol may include generating an algorithm recommendation message and communicating the message to the orchestrator module, the message containing an indication of a recommend one or more of the inference algorithms to load. The message may additionally contain a timing recommendation indicative of recommended timings for starting/stopping the specified algorithm. The orchestrator module may then handle the recommendation message by generating and sending an algorithm request message to the algorithm monitoring module for requesting loading of the algorithm and may control pushing of the algorithm to the algorithm hosting platform in accordance with the timing recommendations.

By way of another example, a protocol specification may indicate that a defined medication is needed at a particular stage. The protocol hosting platform may communicate this to the orchestrator module 22 which may pre-fill a medication prescription request for communication to the prescription ordering platform 72.

Any steps automatically executed by the protocol hosting platform may be communicated to the clinician user via the user interface 62. The user interface may include functionality permitting a user to intervene to stop execution of a certain step of modify its execution.

In some embodiments, a protocol may require certain patient data in order to operate, such as clinical parameter measurement data, e.g. physiological parameter measurements or other clinical parameter measurements. For example, one or more of the steps of the protocol specification may vary in dependence upon values of one or more clinical parameters. For example, the protocol may include a branching logic structure, wherein different branches are the protocol specification are to be followed in dependence upon values of patient clinical parameters. The required input patient data for running the protocol may be obtained by the protocol hosting platform from one or more patient data sources, for example a patient monitoring subsystem which collates (e.g. real-time) physiological and other clinical parameter data for the patient. Another source may be a patient medical record database (e.g. a PDMS 56), which may for example record lab results or more static variables such as weight, age, demographic information, medical history. The required patient data for running the protocol may be referred to as the clinical parameter data requirements for the protocol.

In some embodiments, if the required input clinical parameter data for running the protocol is not available, a data collection sub-routine may be run for acquiring supplementary data to supplement available data. The data collection sub-routine may include: sending to the orchestrator module a data collection request message including an indication of recommended supplementary data to be acquired. The orchestrator module may be adapted to communicate with a data collection sub-system to request acquisition of the supplementary data. The data collection sub-system may for example be a patient monitoring subsystem in some examples. Alternatively, the orchestrator module may generate a prompt at the user interface to prompt a user to take steps to acquire the needed data, or to enable the patient monitoring subsystem to acquire the data (e.g. by installing a needed measurement device such as a central venous line).

The data collection sub-routine may be executed in some embodiments by the protocol module. It may be executed responsive to receipt of a protocol request message from the orchestrator module.

In addition to required input patient data for running a protocol, also there may be required patient personalization data for configuring a protocol, e.g. tuning or adjusting parameters of the protocol. For example, to take the above-mentioned example of a branching or conditional logic structure for the protocol, the personalization data may be used to configure the one or more thresholds or rules to be used to determine which branch to follow (e.g. normal vs pathological threshold levels), while the patient clinical parameter data may be used during execution of the protocol to assess each rule or threshold, i.e. comparing the parameter values against the rules or thresholds.

Thus, in summary, the complexity of the protocol hosting platform may range from displaying the static protocol to a user as a viewable document, e.g. a PDF, to a dynamic system that tracks, records and feeds back protocol progress.

As mentioned above, the orchestrator module 22 is adapted to receive risk messages indicative of a clinical risk.

The orchestrator module 22 may in at least some embodiments be adapted to receive said risk messages or alerts from any one or more of: the system used to admit a patient, the algorithm hosting platform 54, the protocol hosting platform 52, the protocol module 34 and the user interface 62.

By way of brief preliminary summary, the aforementioned orchestrator module 22 may according to at least some embodiments perform one or more of the following general functions: Maintain the interaction between algorithms, protocols, and the monitoring of a patient management environment such as patient monitors, electronic medical record systems (EMR), ventilation systems, laboratory systems, and infusion pumps.
Mediate triggers initiated by algorithms or protocols, such as risk alerts or messages.

Schedule the triggering of protocols and/or algorithms and configure their priority of triggering.

Ensure that the inference algorithms and/or dynamic clinical protocols meet specific operational conditions. This could include validating that they are being used with the correct patient population, they have the necessary input data, and all required patient information fields have been prepopulated.

Ensure that algorithms and protocols are ready for execution before they are executed and made available to the end-user.

The aforementioned algorithm monitoring module 32 may in at least some embodiments perform one or more of the following general functions:
Track available smart analytics, such as inference algorithms.

Specify optimal starting and stopping times of the inference algorithms to the orchestrator module.

Determine a minimum set of information needed to reliably run an algorithm.

If the necessary information is not available, inform the orchestrator module to take actions to ensure this information becomes available by collecting or requesting the necessary information, and/or to prevent the algorithm from becoming active.

Once a system-related trigger has been flagged (such as a measurement has been collected, or a standard operating procedure has been completed), make the algorithm available through the orchestrator module to an algorithm hosting platform, wherein the algorithm hosting platform makes the algorithm available to the end-user.

In situations where certain boundary conditions are not met or will not be met, withhold the algorithm from the user to prevent confusion and additional cognitive burden.

The aforementioned protocol module 34 may in at least some embodiments perform one or more of the following general functions:
keep track of available protocols.
ensure the operability of these protocols when needed.
to prevent delays in operationality, ensure that the already available data is collected and incorporated before making the protocol available.

Determine a minimum set of information needed to reliably configure and run the protocol (e.g. patient personalization data and/or patient measurement data).

If the necessary information is not available, inform the orchestrator module to take action to ensure this information becomes available by collecting or requesting the necessary information. make protocols available to the protocol hosting platform, via the orchestrator module, once all necessary data has been incorporated, wherein the protocol hosting platform makes the protocol available to the end-user.

As discussed above, the system includes two repositories (e.g. datastores) that maintain the clinical workflow protocols and clinical analytics-based monitoring (inference) algorithms. Protocol specifications for each protocol are stored in the protocol datastore 44. The inference algorithms are stored in the algorithm datastore 42.

The system may use metadata contained in these repositories 42, 44 to:
(1) determine which algorithms are available, and with what operating requirements/constraints;
(2) determine what protocols are available, what data is needed to make them operational and under what boundary conditions, and what (complication) risks are associated with steps of the protocol; and/or
(3) determine how the protocols and monitoring algorithms are connected and interact, i.e. trigger events or circumstances which may occur in execution of one algorithm or protocol which logically prompt execution, termination or adjustment of another algorithm or protocol.

Further details relating to the implementation of the orchestration module in accordance with at least a subset of embodiments, will now be described.

As discussed above, the orchestrator module 22 orchestrates when the inference algorithms and the clinical protocols are executed and when they are terminated via the designated platforms (i.e. algorithm hosting platform 54 and the protocol hosting platform 52).

This orchestration may be mediated by system alerts or messages generated by running algorithms or protocols, for example risk messages generated by a running algorithm responsive to a certain metric or score passing a risk threshold, or a running protocol indicating a risk associated with a certain protocol step.

With regards to the execution of algorithms and protocols, another functionality of the system is ensuring the operating requirements for any algorithm or protocol are met before the algorithm or protocol is run.

It is proposed to utilize to assist in this a dedicated protocol module 34 and algorithm monitoring module 32 to manage available protocols and algorithms and preferably to check running requirements are met.

As mentioned previously, the algorithm monitoring module 32 is adapted to: receive a request message for access to one of the inference algorithms, assess a predefined one or more release conditions for the inference algorithm, and generate a release flag/trigger for a given algorithm if the predefined one or more release conditions are met.

As also mentioned previously, the protocol module 34 is adapted to: receive a request message for preloading one of the clinical protocols, and generate a protocol loading package comprising the protocol specification for the specified protocol.

The orchestrator module 22 may be adapted to: receive release flags generated by the algorithm monitoring module 32 responsive to a sent request message relating to a specified algorithm; and push released algorithms to the algorithm hosting platform 54 for execution.

The orchestrator module 22 may be adapted to: receive protocol loading packages from the protocol module, and push protocol specifications comprised by received protocol loading packages to the protocol hosting platform 52.

With regards to managing the triggering of algorithms and protocols at appropriate times, as discussed previously, this may be managed by triggers such as risk messages communicated from various sources such as: a patient admissions system, the algorithm hosting platform 54, the protocol hosting platform 52, the protocol module 34 and a user interface 62.

When a risk message or other trigger is received, the orchestrator module 22 may consult a database 24 which records associations between risk events and triggers and appropriate responses. In other words, the connectivity patterns between risks or triggers, and protocols and monitoring algorithms are maintained and stored and utilized by the orchestrator module.

For example, as mentioned above, the system 20 can comprise risk database 24, recording, for each of a plurality of different clinical risks, one or more suitable clinical protocols and/or inference algorithms for responding to the risk. In some examples, this may for instance take the form of a look-up table. In some examples, this may for instance take the form of a look-up table of algorithms and protocols, their corresponding system triggers, and optionally how the protocols and algorithms are connected.

The suitable clinical protocols and/or inference algorithms may be recorded in the risk database as indications of suitable types or classifications of clinical protocols and/or inference algorithms and wherein the orchestrator module is further configured to search the protocol datastore 44 and/or algorithm datastore 42 to identify a specific protocol or algorithm which matches the target classification or type. Instead of classification or type, also suitable protocols or algorithms may be recorded in terms of descriptors or labels or specifications, and wherein lookup in the protocol or algorithm datastore is based on the descriptors or labels or specifications. Alternatively, the suitable protocols and/or algorithms may be recorded as references or pointers to specific protocols or algorithms available in the protocol datastore 44 and the algorithm datastore 42. For instance, this look-up table connects the (complication) risks of a certain protocol to a specific monitoring algorithm that can monitor that condition. A combination of the two approaches is also possible.

In some embodiments, the orchestrator module 22 may include functionality for ranking the support in order of criticality, urgency, or availability. Some support needs to be available immediately. For instance, resuscitation protocols cannot wait until all personalization data has become available before being run. The system suffices with information currently available and pushes the protocol towards the protocol hosting platform 52. For other protocols (e.g. discharge, ventilator weaning, medication management protocols), the orchestrator 22 may try to gather or promote the collection of measurements (e.g. through making dedicated standard operating procedures available) such that the algorithm or protocol can immediately be put in practice when it is made available to the HCP.

In some embodiments, the orchestrator module 22 may facilitate and promote the collection of information for personalizing and contextualizing protocols. It may also facilitate the background creation of historical measurements that can be used for trending and other data interpretation. As such, the orchestrator module establishes a connection between algorithms and protocols by keeping track of the running processes at a given moment in time. Based on internal or external triggers, the module identifies the need, type (i.e. protocol, monitoring algorithm, measurement data, intervention / medication ordering,), and criticality of the requested support.

In some embodiments, the algorithm datastore 42 further stores, for each of at least a subset of the inference algorithms, metadata which includes an indication of an optimal timing for starting and/or stopping the algorithm. The orchestrator module 22 may be further adapted to control timing of pushing each of the at least subset of algorithms in dependence upon the optimal timing information in the metadata for the respective algorithm.

For example, optimal start and stop times could be determined in advance based on scientific evidence, or based on clinician judgment or experience, or based on hospital best practice.

In some embodiments, the optimal timings for start and/or stop may be adaptive. For example, the timings may be varied in a controlled way in dependence upon a current scenario or situation. For instance, when two protocols describe monitoring for ventilator-associated pneumonia (VAP); one describes the start of monitoring only after 42 hours and the other within 8 hours after admission. The latter might be prioritized. Around 8 hours after admission, the orchestrator module will make the algorithm available to the algorithm hosting platform to start the VAP monitoring.

In some embodiments, stop times of algorithms may be determined based on an output of the algorithm itself or based on an output of another algorithm. For example, if an algorithm generates an output risk score indicative of risk of a certain adverse event or outcome, termination of the algorithm might be performed responsive to the risk score falling below a certain threshold level.

In some embodiments, stopping of an algorithm may be controlled based on a pre-defined set of rules or heuristics (e.g. adaptable rules, or a threshold), or may be determined based on an AI algorithm which predicts the impact of shutting down a monitoring device or algorithm.

In some embodiments, metadata for each algorithm may prescribe certain information to be logged or stored upon termination of an algorithm or protocol, so as to allow traceability with respect to the decision-making of ending of a monitoring algorithm. This data might be added to the PDMS 56 for example.

Further details relating to the implementation of the algorithm monitoring module 32 in accordance with at least a subset of embodiments, will now be described.

The algorithm monitoring module 32 focuses on managing the availability of inference algorithms, and assessing what preparation is required to ensure (optimal) operation. Clinical monitoring algorithms typically serve a particular purpose and come with specific requirements regarding, for example, patient population, data input and output, and/or timing of usage. The algorithm module 32 maintains these metadata and ensures these are fulfilled prior to operation. While certain properties can be readily verified (e.g. verifying patient type or gender), some may require additional time, or some may not be fulfilled at all.

For example, certain algorithms might be flexible or adaptable in terms of their required input data. For example, an algorithm might be operable with a limited set of four data inputs, but have most optimal performance when operated with a greater number of data inputs (e.g. at most 32). However, measurements have limited validity. Vital signs measurements cannot be older than 2 hours and laboratory readings should not be older than 24 hours.

In such a scenario, the algorithm monitoring module may release the given algorithm for use, but communicate a message to the orchestrator module indicating the additional data inputs that would improve quality of outputs of the algorithm. The orchestrator module 22 may then promote the collection of the additional data- if possible- by enabling a standard operating procedure (SOP) to acquire addition data e.g. laboratory readings.

In some situations, certain requirements may be impossible to fulfil. For instance, an MRI cannot be acquired during the night. To prevent confusion or suboptimal operation, the algorithm requiring this information may thus not be released by the algorithm monitoring module to the orchestrator module 22.

As soon as operating requirements are fulfilled the algorithm monitoring module 32 releases the algorithm by announcing its availability to the orchestrator. For example a release flag is generated.

In some embodiments, release of an algorithm may further be communicated to an end-user via the user interface.

Thus, following from the above considerations, as a more general principle, it is proposed in accordance with at least some embodiments, that the algorithm datastore further stores, for each inference algorithm, metadata indicating required input data for running the algorithm. Responsive to receipt of a request message relating to an algorithm, the algorithm monitoring module is further adapted to: read the metadata for the relevant algorithm in the algorithm datastore; determine whether the required input data for the algorithm is currently available; and generate the release flag/trigger for the algorithm only if the input data requirements are met.

In some embodiments, if the input data requirements are not met, the algorithm monitor module is further adapted to execute a data collection sub-routine for acquiring supplementary data to supplement the available input patient data.

For example, in some embodiments, the data collection sub-routine includes: sending to the orchestrator module a data collection request message including an indication of recommended supplementary data to be acquired to supplement the currently available input patient data; and wherein the orchestrator module is adapted to communicate with a data collection sub-system, e.g. patient monitoring sub-system, or an ordering system for ordering diagnostic scans or laboratory tests, to request acquisition of the supplementary data.

In some examples, the data collection sub-routine includes: sending a protocol recommendation message to the orchestrator module, recommending execution of a protocol, wherein the recommended protocol includes steps for acquiring at least a subset of the required input information for the algorithm which is currently not available.

In some embodiments, algorithm datastore further stores, for each inference algorithm, associated metadata including a patient population type for which the algorithm is trained to be applied. Responsive to a request message, the algorithm monitor module may be adapted to: read the metadata for the algorithm in the algorithm datastore; determine whether a patient associated with the input patient data matches the patient population type; and generate a release flag/trigger for a given algorithm only if the patient population type requirements are met.

In some embodiments, the algorithm monitor module is adapted to execute background operations during running of the system comprising, for at least a subset of the algorithms in the algorithm datastore, monitoring whether the required input data for each algorithm is currently available and, if the required input data is not available, executing a data collection sub-routine for acquiring supplementary data to supplement the currently available input patient data. Thus, required data for running an algorithm are compiled in the background before the algorithm is actually needed, thereby ensuring that, if and when the algorithm is requested, the data required for running the algorithm is available immediately.

Further details relating to the implementation of the protocol module 34 in accordance with at least a subset of embodiments, will now be described.

The protocol module 34 maintains the set of protocols available to the system. The protocol module 34 receives requests from the orchestrator module 22 for loading of a particular protocol and generates a loading package containing a data representation of a specification for the protocol for passing to the orchestrator module.

In some embodiments, the protocol module 34 may perform checks to ensure that necessary information for personalizing and operationalizing a protocol is obtained prior to executing the protocol.

For example, in some embodiments, the protocol datastore 44 may further store, for each protocol, metadata indicating: required patient data for configuring and/or running the protocol. Responsive to receipt of a request message, the protocol module 34 may be adapted to: read the metadata for the specified protocol to identify the required patient data.

The metadata may include an indication of required patient personalization data for configuring the protocol. The metadata may include an indication of required patient clinical parameter data for use in running the protocol.

The protocol module may access a patient data source to retrieve the required patient personalization data. The source may be a patient medical record database for example (e.g. a PDMS 56). The protocol module may be adapted to configure the protocol in the protocol package with the patient personalization data.

The protocol module may in some embodiments be adapted to determine whether the required clinical parameter data for a specified protocol is currently available, and if the required clinical parameter data is not available, execute a data collection sub-routine for acquiring supplementary data to obtain the required clinical parameter data. The data collection subroutine may for example comprise communicating with a patient monitoring subsystem 64 to request the supplementary data. The data collection subroutine may comprise generating a prompt message at the user interface 62 to prompt a user to take steps to enable the supplementary data to be collected, for example by attaching or installing a measurement device to a patient and linking it with a patient monitoring subsystem.

With regards to the personalization data, examples of personalization data may include for example any one or more of: patient demographic information, patient medical history, comorbidities, past interventions, vital signs, predictive algorithm outputs, alarm settings (e.g., an upper and/or lower alarm threshold, e.g. 90 mmHg<SBP< 140 mmHg, SpO2>94%), any score or probability related to high risk of death (e.g. GCS, APACHEII, SAPSII, SOFA), ICU mortality, hospital mortality, 90 days mortality or ICU length of stay.

For instance, in a sepsis protocol, if it is known that a patient has COPD, then the oxygen administration step may be triggered at an SpO2 value that is lower than that for which it may be triggered for a patient belonging to the healthy patient population. Thus, for example, this step in the protocol may be made conditional for the COPD patient upon a measured SpO2 value for the patient which is slightly lower, e.g. below 90%.

By way of example, one or more steps of at least one protocol may define steps to be performed which are characterized by quantitative variables, e.g. thresholds for physiological or other clinical parameter measurements to be met before execution of the step, dosage for drugs, time duration for administration of a defined intervention, and/or timings for physiological parameter measurements. These one or more quantitative variables may be configurable in dependence upon pre-defined elements of patient personalization data.

In some cases, the patient personalization data may be essential to the safe implementation of a certain step, e.g. for drugs, a patient weight may be essential to determine the correct dose. In other cases, if needed patient personalization data is not available for personalizing a particular step of a protocol, a generic or population average value for the variable may be used. For instance, examples of population-generic workflow steps might include: "Give fluid challenge with lactated ringers (1L in 30 min.). Minimally 30ml/kg in 3 hours if hypotensive or lactate>2", or "Monitor blood pressure every 10 min. to assess the effect of the fluid challenge." "Aim for MAP of 65 mmHg if vasopressors are needed in septic shock."

Where relevant patient personalization data is available (as defined in the metadata for the protocol), the numerical variable can be made more exact to match better to the current patient state.

With regards the patient clinical parameter data, this could include for example vital signs or other physiological parameters. It may include clinically specific measurement data. By way of just one illustrative example, in a sepsis monitoring protocol, certain steps may conditionally depend upon a value for ventral venous pressure (CVP), which is measured by a central venous line. The sepsis monitoring protocol may therefore include in its metadata an indication that CVP data is needed to run the protocol. The protocol module 34 may therefore check whether CVP data is currently available. If not, a data collection sub-routine may be triggered. This may comprise for example querying a patient monitoring subsystem 64 to determine whether this data is available to the patient monitoring subsystem. If not, a message may be generated at the user interface 62 to trigger a health care personnel to place a central venous line. This way, once the protocol is pushed to the protocol hosting platform 52, the CVP data will already be available.

In some embodiments, physician profile information may in addition be used to personalize the protocol. It is common that different physicians will implement certain protocol steps in different particular ways. Physician profile information may include user input from a physician specifying one or more preferences regarding one or more steps of a protocol. Alternatively, physician profile information may be inferred automatically from historical data (if available) recording a manner in which a physician implemented a protocol step in the past.

In some embodiments, the protocol datastore 44 may further store, for each protocol, metadata indicating any further clinical risk associated with any one or more steps of the protocol. Responsive to receipt of a request message, the protocol module 34 may be further adapted to read the metadata for the specified protocol to identify any further clinical risk associated with any one or more steps of the protocol, and wherein the protocol loading package further includes the retrieved further clinical risk information associated with the relevant protocol.

Thus, in other words, for each protocol, the protocol datastore 44 may further record any one or more medical risks that certain protocol (steps) may entail. The risk database 24 may define a risk incurred by a protocol step and an algorithm that allows for monitoring that condition. This connection can be used by the orchestrator module to initiate the monitoring of that risk through an algorithm. For example, an intubated patient runs the risk of developing ventilator-induced pneumonia (VAP) which may culminate in acute respiratory distress syndrome (ARDS). Within the intubation protocol, it can be advised to monitor the patient closely for VAP. This monitoring algorithm can be preloaded by the system and made available to the user via the algorithm hosting platform 54. This can be done either automatically or the user is asked for confirmation to keep the caregiver in control of the process.

To provide further understanding of proposed inventive concepts, there will now be described a first example scenario in which a system 20 according to at least one set of embodiments is implemented to monitor risk of complications through coordinated management of a clinical protocol and inference algorithm. This scenario is presented for illustrative purposes only, to exemplify an implementation of the system, and features of this example are not necessarily essential to the general inventive concept.

In this example scenario, a patient is admitted to an ICU unit following Coronary Artery Bypass Grafting (CABG) surgery. CABG is a major surgery and patients will often require a period of intensive care postoperatively. These patients need to be monitored closely for complications such as infection, arrhythmias, poor heart function, or other issues related to the surgery.

In the following scenario, for simplicity, working of the system for managing risk of just one possible complication is discussed, namely the risk of ventilator-associated pneumonia (VAP) due to prolonged intubation.

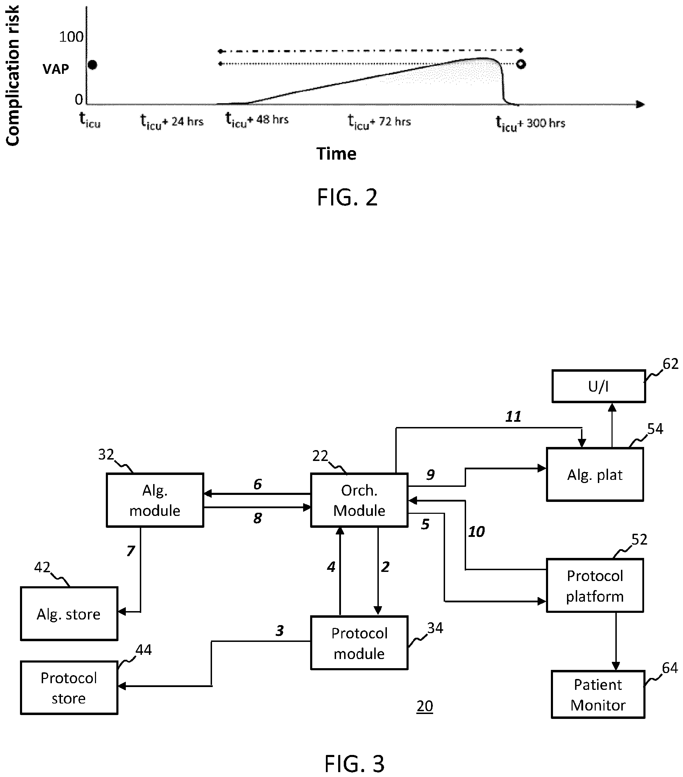

Fig. 2 shows a graph which illustrates how VAP risk develops over time post admission to ICU. The y-axis of the graph represents complication risk level, and the x-axis indicates time post admission.

The risk of VAP typically only begins to arise after more than 48 hours of mechanical ventilation. After this time, it progressively increases as a function of time, up to 300 hours post admission, at which point it falls away.

In this scenario, the protocol datastore 44 is assumed to contain a dedicated protocol for monitoring VAP risk.

The operations of the system in relation to VAP risk begun upon admission of the patient to ICU, and are schematically illustrated the block diagram of Fig. 3. The diagram indicates, with bold numbers, steps implemented by the system in accordance with this example scenario.

Upon admission, a risk message is communicated (step 1) to the orchestrator module by the system used in admitting the patient, e.g. the user interface 62 or the patient monitoring system 64. The risk message indicates a risk of VAP may become critical after 48 hours of ventilation.

The orchestrator module 22, responsive to the received risk message, consults the risk database 24 and identifies a dedicated protocol (VAP protocol) most suitable for monitoring the risk.

The orchestrator module 22 generates and sends a request message (step 2) to the protocol module 34, requesting pre-loading of the VAP protocol.

The protocol module 34 communicates with the protocol store 44 to retrieve the VAP protocol (step 3).

The protocol module 34 then generates a protocol loading package comprising the protocol specification for the VAP protocol and communicates the protocol loading package to the orchestration module 22 (step 4).

The orchestration module 22 receives the VAP protocol loading package from the protocol module, and pushes (step 5) the protocol specification comprised by received protocol loading package to the protocol hosting platform 52.

In this example, the VAP protocol specifies execution of a VAP monitoring algorithm at 42 hours after ICU admission. The protocol hosting platform 52 communicates this element of the protocol to the orchestration module 22. The orchestration module, at the appropriate time, sends a request message (step 6) to the algorithm monitoring module 32 requesting access to the VAP monitoring algorithm. The algorithm monitoring module 32 receives the request message, retrieves the VAP monitoring algorithm from the algorithm datastore 42 (step 7), and makes available the algorithm to the orchestrator module 22. The orchestrator module 22 may then push the released algorithm to the algorithm hosting platform 54 at the appropriate time (step 9). In some embodiments the algorithm monitoring module is adapted to assess a predefined one or more release conditions for the VAP algorithm before releasing it, and generating a release flag/trigger for a given algorithm only if the predefined one or more release conditions are met. The orchestrator module 22 may receive such release flags generated by the algorithm monitoring module 32, and push the released algorithm to the algorithm hosting platform 54 at the appropriate time.

The algorithm hosting platform 54 hosts the running of the algorithm. The VAP monitoring algorithm may be adapted to receive input data from one or more physiological parameters, and is adapted to output a VAP risk score indicative of a risk or probability of VAP.

For example, the one or more physiological parameters input to the algorithm might include one or more of:
- respiratory parameters: mechanical ventilation settings such as tidal volume, positive end-expiratory pressure (PEEP), and/or other variables.
- oxygenation data: Oxygen saturation (SpO2), arterial oxygen partial pressure (PaO2), and/or the ratio of arterial oxygen partial pressure to fractional inspired oxygen (PaO2/FiO2).
- temperature: Fever can be an early sign of infection, and continuous temperature monitoring could contribute to a VAP prediction algorithm.
- Heart Rate and Blood Pressure: Changes in vital signs can indicate an underlying infection.
- End-tidal CO2: Changes in end-tidal carbon dioxide levels could signal issues with ventilation-perfusion matching, which can occur in pneumonia.
- Patient position: Ventilated patients are often positioned in a way (like semi-recumbent) to reduce the risk of VAP. Monitoring patient position could be relevant if this information can be collected via sensors.

The VAP protocol specification indicates that VAP risk dissipates after a patient has been successfully weaned from ventilation after 300 hours. Thus, 300 hours after admission, the protocol platform 52 which hosts the VAP protocol may send a message (step 10) to the orchestrator module 22 indicating that the protocol has been completed. Responsive to this, the orchestrator module 22 communicates with the algorithm hosting platform 54 (step 11) to terminate execution of the VAP monitoring algorithm.

To provide further understanding of proposed inventive concepts, there will now be described a second example scenario in which a system 20 according to at least one set of embodiments is implemented to monitor risk of complications through coordinated management of a clinical protocol and inference algorithm. This scenario is presented for illustrative purposes only, to exemplify an implementation of the system, and features of this example are not necessarily essential to the general inventive concept.

This second scenario is more clinically complex and requires a more orchestrated interaction between components of the system.

As in the first scenario, this scenario also follows a patient admitted to an ICU unit following Coronary Artery Bypass Grafting (CABG) surgery. However, a plurality of possible risks are monitored and managed rather than just one. This might be done in addition to or instead of the management of VAP discussed in scenario 1.

In this scenario, risk of each of myocardial infarction (MI), acute kidney injury (AKI) and bleeding are considered.

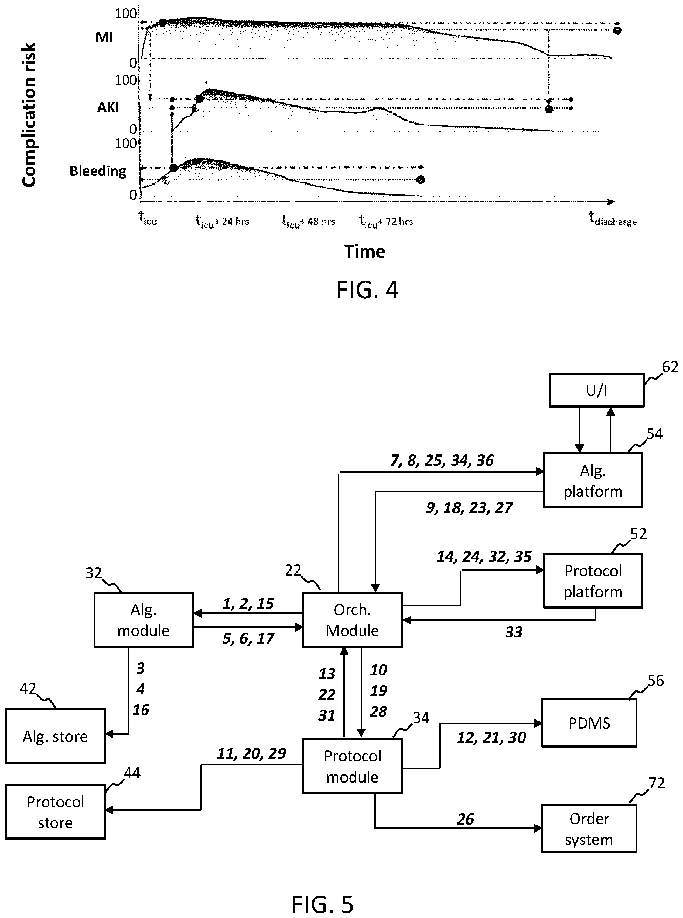

Fig. 4 shows a set of three graphs which illustrate how risk of each of MI, AKI, and bleeding develop over time post admission to ICU. The y-axis of the graph represents complication risk level, and the x-axis indicates time post admission.

The operations of the system in relation to the various risks begin upon admission of the patient to ICU, and are schematically illustrated the block diagram of Fig. 5. The diagram indicates, with bold numbers, steps implemented by the system in accordance with this example scenario.

As is apparent from Fig. 4, in the first hours after ICU admission, the patient has an increased MI and bleeding risk.

This information may be communicated to the orchestrator module 22 as a risk message upon patient admission, e.g. by the system that is used to admit the patient, e.g. a user interface 62. Alternatively, an internal assessment module of the orchestrator module may, based on reviewing the patient medical record, e.g. in the PDMS 56, identify the initial risk factors and self-generate the risk message indicative of the MI and bleeding risk.

In either case, responsive to this, the orchestrator module 22 consults the risk database 24 and identifies two dedicated inference algorithms adapted for monitoring each of MI risk and bleeding risk based on patient sensor data.

The orchestrator module 22 sends a request message (step 1, step 2) to the algorithm monitoring module 32, requesting access to the MI monitoring algorithm and the bleeding risk monitoring algorithm.

The algorithm monitoring module 32 retrieves the two algorithms (step 3, step 4) from the algorithm datastore. 42.

The algorithm module 32 releases the two algorithms to the orchestrator module 22 (step 5, step 6).

The orchestrator module 22 pushes the two algorithms to the algorithm hosting platform 54 (step 7, step 8) to start the monitoring process.

In this example scenario, soon after admission, the MI risk score generated by the MI risk prediction algorithm increases and passes a pre-defined risk threshold. This risk threshold acts as a system trigger.

In particular, the MI algorithm generates a risk alert responsive to the metric or score generated by the algorithm passing a risk threshold, and the algorithm hosting platform communicates this to the orchestrator module 22 as a risk message or system trigger (step 9).

Responsive to this received risk message, the orchestrator module 22 may consult the risk database 24 again and identifies an appropriate monitoring protocol (MI protocol) for responding to the MI risk alert. The orchestrator module sends a request message (step 10) to the protocol module 34 requesting pre-loading of the identified MI protocol.

The protocol module 34 communicates with the protocol datastore 44 to retrieve a protocol specification for the MI protocol (step 11). The specification includes metadata which specifies required patient personalization data for running the protocol and also includes metadata indicating any further clinical risk associated with any one or more steps of the protocol.

The protocol module 34 reads the metadata for the MI protocol to identify the required patient personalization data and the additional risk data.

With regards to the personalization data, the protocol module automatically accesses a patient database 56 to retrieve the required patient personalization information from the patient database (step 12), and configures the protocol in the protocol package with the patient personalization data. The personalization data in this case may for example comprise demographic information such as age and gender and also medication information.

With regards to additional clinical risks, the metadata indicates that one of these risks is acute kidney injury (AKI). This information is included as a risk message in a protocol loading package along with the protocol specification and this package is then sent (step 13) to the orchestrator module 22. The orchestrator module 22 receives the protocol loading package and pushes the MI protocol specification to the protocol hosting platform 52 (step 14).

Responsive to the additional risk message in the protocol loading package, the orchestrator module 22 consults the risk database 24 again and identifies an inference algorithm suitable for monitoring the AKI risk. The orchestrator module sends a request message (step 15) to the algorithm monitoring module 32 request access to the AKI monitoring algorithm.

The algorithm monitoring module 32 retrieves (step 16) the AKI monitoring algorithm from the algorithm datastore 42. The algorithm monitoring module releases the AKI algorithm to the orchestrator module 22 (step 17). The orchestrator module may begin collecting input data for the algorithm so as to acquire a historical trend which the algorithm can employ once it is pushed by the orchestrator module to the algorithm hosting platform 54. This may be referred to as preloading of the algorithm.

In the meantime, the (already running) bleeding algorithm detects an increased risk of bleeding, for example, the bleeding risk score generated by the algorithm exceeds a pre-defined risk threshold. Responsive to this, the bleeding algorithm sends a risk message or alert to the orchestrator module 22 (step 18).

Responsive to this bleeding risk message, the orchestrator module 22 consults the risk database 24 again and identifies an appropriate protocol for monitoring and managing the bleeding risk. The orchestrator module 22 sends a request message (step 19) to the protocol module 34 requesting preloading of the bleeding protocol. The protocol module 34 retrieves a protocol specification for the bleeding protocol from the protocol datastore 44 (step 20), and prepares a protocol loading package containing the protocol specification. The protocol module further reads metadata of the protocol specification indicating required patient personalization data, which is then retrieved from a patient database 56 (step 21) and used to configure or personalize the protocol accordingly, e.g. by filling in the blood group, age, gender, known comorbidities. The protocol module may further check whether any steps of the protocol have already been executed.

The protocol module 34 also includes in the protocol loading package information about any additional risks associated with any of the steps of the protocol.

The bleeding protocol loading package is communicated to the orchestrator module (step 22).

Shortly after, the bleeding algorithm sends an alert risk message (step 23) to the orchestrator module 22 indicating that a second (higher) risk threshold has been passed. Responsive to this, the orchestrator module now pushes the pre-loaded bleeding protocol to the protocol hosting platform (step 24), and also may be presented or otherwise made available at the user interface 62 for a clinician user to observe. At the same time, triggered by the same risk alert message, the orchestrator module pushes the AKI risk algorithm to the algorithm hosting platform 54 (step 25).

Responsive to a recommendation message received from the bleeding protocol, the orchestrator module 22 also prepares a prescription request for packed red blood cells (PRBC) - an intervention required to resolve the situation - and forwards this to a prescription request platform 72 (step 26). Alternatively, this step could be triggered by a received user input.

The clinician user might review the request on the user interface 62 before it is forwarded.

An output risk score of the AKI risk monitoring algorithm is presented to the clinician user on the user interface 62.

The administration of PRBC causes an increase in AKI risk score, as computed by the AKI risk algorithm. This triggers communication of a risk message (step 27) to the orchestrator module. This triggers the retrieval of the AKI protocol by the orchestrator that makes it available to protocolhosting platform. This may be triggered for example by the orchestrator module consulting the risk database, or based on an indication from the bleeding protocol, or based on an input from a user interface.

To this end, the orchestrator module 22 sends a request message to the protocol module 34 requesting pre-loading of the AKI protocol (step 28). The AKI protocol specification is retrieved from the protocol datastore 44 (step 29). The protocol module retrieves required patient personalization data for the protocol from the patient database 56 and configures the protocol accordingly (step 30). The protocol specification is then communicated in a protocol loading package to the orchestrator module (step 31). The orchestrator module 22 then pushes the AKI protocol to the protocol hosting platform 52 (step 32).

The AKI protocol prescribes administering of renal replacement therapy. The clinician user follows this and commences renal replacement therapy. This leads to reduction of the risk.

After a few hours, the risk for MI, bleeding and AKI have dissipated.

The orchestrator 22 is notified (step 33) by the protocol platform 52 that the bleeding protocol is completed. Responsive to this, the orchestrator module notifies the algorithm hosting platform 54 to terminate execution of the bleeding algorithm (step 34).

The low risk in MI reduces the need for monitoring and managing of AKI. The orchestrator 22 initiates the termination of the AKI protocol (step 35) and thus the renal replacement therapy.

The AKI monitoring algorithm is terminated (step 36) is stopped shortly after proper renal function has been verified. The MI monitoring continues till discharge at which all monitoring activities are terminated.

To provide further understanding of proposed inventive concepts, there will now be described a third example scenario in which a system 20 according to at least one set of embodiments is implemented to monitor risk of complications through coordinated management of a clinical protocol and inference algorithm. This scenario is presented for illustrative purposes only, to exemplify an implementation of the system, and features of this example are not necessarily essential to the general inventive concept.

This third scenario demonstrates how the orchestrator module 22 may handle monitoring of risks from different protocols.

As in the first and second scenario, this scenario also follows a patient admitted to an ICU unit following Coronary Artery Bypass Grafting (CABG) surgery. In this scenario, risk of each of bleeding, embolus/stroke and atrial fibrillation (AF) are monitored.

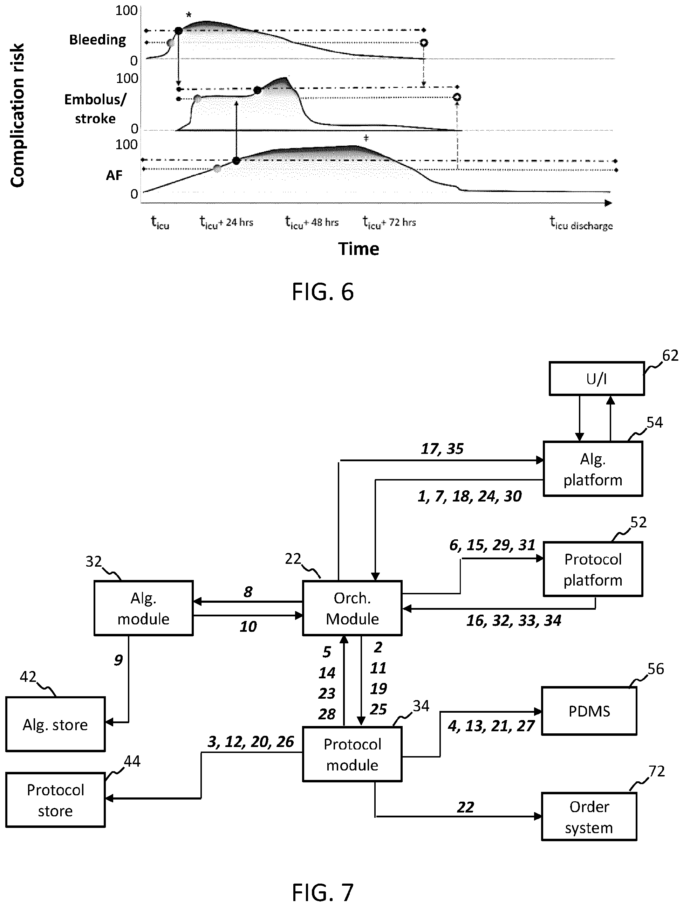

Fig. 6 shows a set of three graphs which illustrate how risk of each of bleeding, embolus/stroke, and atrial fibrillation (AF) develop over time post admission to ICU. The y-axis of the graph represents complication risk level, and the x-axis indicates time post admission.

The operations of the system in relation to the various risks begin upon admission of the patient to ICU, and are schematically illustrated the block diagram of Fig. 7. The diagram indicates, with bold numbers, steps implemented by the system in accordance with this example scenario.

This scenario assumes that a bleeding risk algorithm and AF risk algorithm is already running at the algorithm hosting platform, which are adapted to generate a risk score indicative of risk of bleeding and a risk score of AF respectively.

The bleeding risk score exceeds a first risk threshold, triggering a risk message to be sent to the orchestrator module 22 (step 1). Responsive to this, based on reference to the risk database 24, the orchestrator module 22 identifies an appropriate protocol for managing the bleeding risk. The orchestrator module sends a request message (step 2) to the protocol module 34, requesting the bleeding protocol. The protocol module retrieves the bleeding protocol specification (step 3) from the protocol datastore 44. The protocol module retrieves (step 4) patient personalization data for the protocol from a patient datastore 56 and configures the protocol accordingly. The protocol module prepares a protocol loading package including the protocol specification and also information about any additional risks associated with any of the steps of the protocol. This bleeding protocol package is sent (step 5) to the orchestrator module. The bleeding protocol is pushed (step 6) to the protocol hosting platform 52.

Some time following this, a second (higher) risk threshold is passed by the bleeding risk score generated by the bleeding algorithm, this triggering a further risk message or alert to be sent by the risk algorithm to the orchestrator module 22 (step 7).

Responsive to this, the bleeding protocol specifies that a stroke monitoring algorithm should be run.

Accordingly the orchestrator 22 module sends a request message (step 8) to the algorithm monitoring module 32 requesting access to a stroke monitoring algorithm. The algorithm module 32 retrieves the stroke monitoring algorithm (step 9) and releases it for use by the orchestrator module (step 10).

In addition, a stroke standard operating procedure (SOP) is also recommended by the bleeding protocol, and so the orchestrator module sends a request message (step 11) to the protocol module requesting pre-loading of the stroke SOP. The protocol module retrieves the stroke SOP specification (step 12), retrieves personalization data for the SOP from a patient database 56 (step 13) and generates a loading package including the SOP specification. An SOP for purposes of the present disclosure is a category of protocol.

The stroke SOP package is communicated to the orchestrator module 22 (step 14) and the stroke SOP is pushed to the protocol hosting platform (step 15). The SOP may include specification for example to acquire a CT scan and/or to acquire a National Institutes of Health Stroke Scale (NIHSS) score.

Once the stroke SOP is complete, this is communicated to the orchestrator module 22 (step 16).

The orchestrator module 22 then pushes the already released stroke risk monitoring algorithm to the algorithm hosting platform 54 (step 17).

The stroke risk score generated by the stroke monitoring algorithm passes a first risk threshold and a corresponding risk message is communicated to the orchestrator module 22 (step 18). This triggers the orchestrator module 22 to send a request message (step 19) to the protocol module 34 requesting pre-loading of a stroke risk protocol. The protocol module retrieves the stroke protocol specification (step 20), retrieves personalization data for the protocol from a patient database 56 (step 21) and generates a loading package including the protocol specification and information about any risks associated with any steps of the protocol. Furthermore, the protocol module prefills a medication order (step 22) by shortlisting antithrombic medication options in the ordering system (e.g. recombinant-tissue plasminogen activator, heparin, statins). The stroke protocol package is communicated to the orchestrator module 22 (step 23).

The AF risk score generated by the monitoring algorithm exceeds a risk threshold and a risk message is accordingly communicated (step 24) to the orchestrator module. Responsive to this, the orchestrator module 22 sends a request message to the protocol module 34 requesting an AF protocol. The protocol module retrieves the AF protocol specification (step 26), retrieves personalization data for the AF protocol from a patient database 56 (step 27) and generates a loading package including the AF specification and information about any risks associated with any steps of the protocol. The AF protocol package is communicated to the orchestration module 22 (step 28). The AF protocol is pushed to the protocol hosting platform (step 29).

The AF protocol prescribes monitoring ischemic stroke. However, since stroke monitoring was already initiated by the bleeding management protocol, the orchestrator 22 ignores the request. With the ever-increasing risk of AF (see Fig. 6), the risk of stroke becomes critical and a stroke risk alert message is sent to the orchestrator module 22 (step 30). Responsive to this, the orchestrator module pushes the stroke management protocol to the protocol hosting platform (31).

The health care practitioner is advised by the system, via control of the user interface 62, of the loading of the stroke management protocol and of the output of the stroke monitoring algorithm. Using this information, the HCP can quickly act by updating the medication order and submitting it. The situation can now be quickly resolved and the stroke risk quickly decreases.

With the bleeding risk dissipating after a couple of hours, the bleeding management protocol can be closed (step 32). This also means that from the bleeding management perspective the stroke monitoring can also be stopped (step 33). However, since the AF risk still requires monitoring of stroke, the orchestrator module 22 keeps the stroke risk monitoring algorithm operational until the AF management protocol prescribes to stop monitoring for stroke (step 34, step 35). The AF monitoring continues till discharge, at which point all monitoring activities are terminated.

In some cases, a given monitoring algorithm may require additional measurements to function effectively. For instance, in the case of Hemodynamic Stability Index (HSI), optimal operation is achieved when all 32 parameters are gathered within a valid time frame. Although the algorithm can accommodate fewer measurements, its reliability increases when all parameters are available. Implementing a Standard Operating Procedure (SOP) to collect more recent lab results before the algorithm is activated could 1) enhance the effectiveness of the algorithm, and 2) reduce the time lapse between the algorithm becoming operational and the arrival of measurements. The system can even autopopulate the lab order, only requiring the healthcare provider (HCP) to review or confirm the order.

Regarding the prioritization of algorithm or protocol system triggers, alongside the predefined system triggers and alarm settings for each individual algorithm stored in the repository, the system can be designed to adapt these triggers. The orchestrator module may track the various active triggers and modify their sensitivity based on the prevailing circumstances. For example, if a systolic blood pressure (SBP) alarm has been activated (i.e., SBP < 90), there would be no need to activate an HSI trigger. Since the HCP has already been alerted to the impending hemodynamic instability, additional alerts or HSI monitoring are redundant. In response, the system can heighten the trigger sensitivity for HSI.

The system can also adjust triggers based on administered treatments. For instance, administering dobutamine elevates heart rate, and algorithms or protocols triggered by increased heart rate might respond even when unnecessary. Adjusting these triggers can prevent unwarranted preparation of additional monitoring or protocols.

Moreover, with the addition of a new parameter - prescribed or not by a protocol or SOP - to the multiparametric prediction, the sensitivity threshold should be adjusted to better predict the condition of interest, such as sepsis, myocardial infarction, hypotension, etc.

Furthermore, the process of triggering, initiating, and stopping an algorithm or protocol can be expanded to consider hospital operations like personnel availability, equipment availability, or other resources. For instance, preparation for surgery can only be initiated when operating rooms are accessible. Ordering a CT scan when there is high demand might delay the protocol initiation. Alternatively, ordering medications that require preparation time (e.g., vasopressors, which may take 30 minutes to reach the ICU) necessitates proactive ordering to ensure timely availability. In such cases, the system adapts by advancing the order, thus ensuring the medication is available sooner.

Embodiments of the system described previously may employ one or more processors. The system may in general comprise a single processor or a plurality of processors. It may be located in a single containing device, structure or unit, or it may be distributed between a plurality of different devices, structures or units. Reference therefore to a module or other component of the system being adapted or configured to perform a particular step or task may correspond to that step or task being performed by any one or more of a plurality of processing components, either alone or in combination. The skilled person will understand how such a distributed processing device can be implemented. The system may include a communication module or input/output for receiving data and outputting data to further components.

The one or more processors of the system can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A clinical decision support system, comprising:
an algorithm datastore storing: a plurality of inference algorithms, wherein each inference algorithm is adapted to receive a set of input patient data and generate an output metric or score indicative of a pre-defined aspect of patient status or for a pre-defined clinical risk;
a protocol datastore storing: a plurality of clinical protocol specifications, each protocol specification comprising a data representation of a clinical protocol associated with monitoring and/or treating a patient in respect of a pre-defined clinical condition or clinical risk, and optionally including one or more machine-executable steps;
an algorithm monitor module adapted to: receive a request message for access to one of the inference algorithms, assess a predefined one or more release conditions for the inference algorithm, and generate a release flag for a given algorithm if the predefined one or more release conditions are met;
a protocol module adapted to: receive a request message for loading one of the clinical protocols, and generate a protocol loading package comprising the protocol specification for the specified protocol;
a risk database, recording, for each of a plurality of different clinical risks, one or more suitable clinical protocols and/or inference algorithms for responding to the risk;
an orchestrator module, adapted to:
receive one or more risk messages or alerts indicative of possible clinical risks;
responsive to receiving a risk message, consult the risk database to identify an inference algorithm in the algorithm datastore suitable for monitoring the relevant risk and/or to identify a protocol in the protocol datastore suitable for managing the risk; and
generate a request message for sending to the algorithm monitor module for requesting access to the identified inference algorithm(s) and/or sending a request message to the protocol module for requesting loading of the identified protocol.

2. The system of claim 1,
wherein the system further comprises an algorithm hosting platform adapted to host and run any of the one or more of the inference algorithms; and
wherein the orchestrator module is adapted to:
receive release flags generated by the algorithm monitoring module responsive to a sent request message relating to a specified algorithm, and
push released algorithms to the algorithm hosting platform for execution.

3. The system of claim 1 or 2,
wherein the system further comprises a protocol hosting platform adapted to host any of the one or more protocols;
wherein the orchestrator module is adapted to:
receive protocol loading packages from the protocol module, and
push protocol specifications comprised by received protocol loading packages to the protocol hosting platform.

4. The system of any preceding claim, wherein the orchestrator module is adapted to receive said risk messages or alerts from any one or more of: the algorithm hosting platform, the protocol hosting platform, the protocol module, and a user interface.

5. The system of claim 4, wherein each inference algorithm is adapted to generate a risk alert responsive to the metric or score generated by the algorithm passing a risk threshold.

6. The system of any preceding claim,
wherein the protocol datastore further stores, for each protocol, metadata indicating: any further clinical risk associated with any one or more steps of the protocol;
wherein, responsive to receipt of a request message, the protocol module is further adapted to read the metadata for the specified protocol to identify any further clinical risk associated with any one or more steps of the protocol; and
wherein the protocol loading package further includes the retrieved further clinical risk information associated with the relevant protocol.

7. The system of any preceding claim,
wherein the protocol datastore further stores, for each protocol, metadata indicating: required patient personalization data for configuring the protocol;
wherein, responsive to receipt of a request message, the protocol module is further adapted to:
read the metadata for the specified protocol to identify the required patient personalization data,
access a patient data source to obtain the required patient personalization data, and
configure the protocol in the protocol package with the patient data.

8. The system of any preceding claim,
wherein the protocol datastore further stores, for each protocol, metadata indicating: required patient clinical parameter data for running the protocol;
wherein, responsive to receipt of a request message, the protocol module is further adapted to:
read the metadata for the specified protocol to identify the required clinical parameter data,
determine whether the required clinical parameter data for the specified protocol is currently available;
if the required clinical parameter data is not available, execute a data collection sub-routine for obtaining the required clinical parameter data.

9. The system of any preceding claim,
wherein the algorithm datastore further stores, for each inference algorithm, metadata indicating required input data for running the algorithm; and
wherein, responsive to receipt of a request message relating to a specified algorithm, the algorithm monitor module is further adapted to:
read the metadata for the relevant specified algorithm in the algorithm datastore,
determine whether the required input data for the specified algorithm is currently available, and
generate the release flag for the algorithm only if the input data requirements are met.

10. The system of claim 9,
wherein, if the input data requirements are not met, the algorithm monitor module is further adapted to execute a data collection sub-routine for acquiring supplementary data to supplement the currently available patient data, and
optionally wherein:
the data collection sub-routine includes: sending to the orchestrator module a data collection request message including an indication of recommended supplementary data to be acquired to supplement the currently available patient data; and
the orchestrator module is adapted to communicate with a data collection subsystem to request acquisition of the supplementary data.

11. The system of claim 10, wherein the data collection sub-routine includes:
sending a protocol recommendation message to the orchestrator module, recommending execution of a protocol, wherein the recommended protocol includes steps for acquiring at least a subset of the required input information for the specified algorithm which is currently not available.

12. The system of any of claims 9-11, or of claim 8,
wherein the algorithm monitor module is adapted to execute background operations during running of the system comprising, for at least a subset of the inference algorithms in the algorithm datastore, monitoring whether the required input data for each algorithm is currently available and, if the required input data is not available, executing a data collection sub-routine for acquiring supplementary data to supplement the currently available data; and/or
wherein the protocol module is adapted to execute background operations during running of the system comprising, for at least a subset of the protocols in the protocol datastore, monitoring whether the required clinical parameter data for running each protocol is currently available and, if the required input data is not available, executing a data collection sub-routine for obtaining the required clinical parameter data.

13. The system of any preceding claim,
wherein the algorithm datastore further stores, for each inference algorithm, associated metadata including a patient population type for which the algorithm is configured to be applied; and
wherein, responsive to a request message, the algorithm monitor module is adapted to:
read the metadata for the algorithm in the algorithm datastore;
determine whether a patient associated with the input patient data matches the patient population type; and
generate a release flag for a given algorithm only if the patient population type requirements are met.

14. The system of any preceding claim, wherein the orchestrator module is further adapted to:
receive a prescription medication recommendation from a running protocol;
fill one or more fields of a medication request form based on the request;
generate an alert on a user interface to alert a user to the medication recommendation;
receive a user input from the user interface indicative of acceptance or rejection of the medication recommendation, and
responsive to acceptance, forwarding the form to a prescription request platform.

15. The system of any preceding claim, wherein the system further includes a patient monitoring sub-system comprising one or more physiological parameter sensors and a patient monitoring control module adapted to receive and compile data from the one or more physiological parameter sensors.
